# EUROPÄISCHE PATENTSCHRIFT

(11) **EP 2 788 309 B1**
(45) Veröffentlichungstag und Bekanntmachung des Hinweises auf die Patenterteilung: **03.02.2016**
(21) Anmeldenummer: 12791101.4
(22) Anmeldetag: 20.11.2012
(51) Int. Cl.: C07C 51/02, C07C 51/44, C07C 51/48, C07C 51/235, C07C 53/126, C07C 53/128

(54) **VERFAHREN ZUR GEWINNUNG VON ALIPHATISCHEN MONOCARBONSÄUREN AUS DESTILLATIONSRÜCKSTÄNDEN**
PROCESS FOR RECOVERING ALIPHATIC MONOCARBOXYLIC ACIDS FROM DISTILLATION RESIDUES
PROCÉDÉ POUR OBTENIR DES ACIDES MONOCARBOXYLIQUES ALIPHATIQUES À PARTIR DE RÉSIDUS DE DISTILLATION

(30) Priorität: 08.12.2011 DE 102011120587
(43) Veröffentlichungstag der Anmeldung: 15.10.2014
(73) Patentinhaber: OXEA GmbH, 46147 Oberhausen (DE)
(72) Erfinder: FREY, Guido, 64560 Riedstadt (DE); ARNOLD, Jörg, 46535 Dinslaken (DE); HÖFS, Wolfgang, 46147 Oberhausen (DE); KRAMER, Matthias, 46244 Bottrop-Grafenwald (DE); MÜLLER, Thomas, 46535 Dinslaken (DE); STRUTZ, Heinz, 47445 Moers (DE)
(86) Internationale Anmeldenummer: PCT/EP2012/004809
(87) Internationale Veröffentlichungsnummer: WO 2013/083236

(56) Entgegenhaltungen:
- DE-C- 950 007
- US-A- 5 504 229

## Beschreibung

Die vorliegende Erfindung betrifft ein Verfahren zur Gewinnung von aliphatischen Monocarbonsäuren aus Destillationsrückständen durch Behandlung dieser Rückstände mit einer wässrigen Säure in einem Rohrreaktor.

Aldehyde sind die gebräuchlichen Ausgangsstoffe zur Gewinnung von Carbonsäuren. Die Vorzugsstellung für dieses Einsatzgebiet verdanken sie ihrer mannigfachen Verfügbarkeit und der leichten oxidativen Umwandlung der Carbonylgruppe in die Carboxylgruppe. Im Rahmen technisch ausgeübter Verfahren erfolgt die Umsetzung von Aldehyden zu Carbonsäuren entweder in Gegenwart oder in Abwesenheit von Katalysatoren oder Zusatzstoffen. Als Katalysatoren kommen vorwiegend Salze von Übergangsmetallen in Betracht, insbesondere Salze des Kobalts und des Mangans sowie des Chroms, Eisens, Kupfers, Nickels, Silbers und Vanadiums. Häufig ist die Carbonsäurebildung aus Aldehyden, selbst bei Einhaltung optimaler Temperaturbedingungen, mit Neben- und Äbbaureaktionen verbunden. Das gilt gleichermaßen für Reaktionen in Gegenwart wie in Abwesenheit von Katalysatoren. In solchen Fällen kann die Selektivität der Umsetzung durch Verwendung von Alkali- oder Erdalkalimetallsalzen schwacher Säuren als Zusatzstoffe erheblich verbessert werden (Ullmanns Encyclopädie der technischen Chemie, 4. Auflage 1975 Band 9, Seite 139).

Besonders bei der Oxidation von aliphatischen, α-alkylverzweigten Aldehyden, bei denen das dem Carbonylkohlenstoffatom benachbarte Kohlenstoffatom die Alkylverzweigung trägt, empfiehlt der Stand der Technik den Zusatz geringer Mengen an Alkalimetallcarboxylaten zur Selektivitätsverbesserung.

So ist beispielsweise aus DE 950 007 bekannt, dass die Oxidation von in α-Stellung verzweigten Aldehyden den Zusatz geringer Mengen von carbonsauren Alkalisalzen erfordert, um die gewünschte Carbonsäure in hoher Ausbeute bei gleichzeitig hoher Reinheit zu erhalten. Aus US 5 504 229 ist bekannt, den bei der Destillation von α-alkylverzweigten Carbonsäuren erhaltenen alkalimetallhaltigen Destillationsrückstand wieder erneut für die Aldehydoxidation zu verwenden. Auch wird beschrieben, aus dem Destillationsrückstand durch Ansäuern die α-alkylverzweigte Carbonsäure freizusetzen. Die anschließend gereinigte Carbonsäure zeigt jedoch nur eine mäßige Farbzahl.

Nach der Lehre der offengelegten japanischen Patentanmeldung 53-105413 oxidiert man aliphatische, α-verzweigte Aldehyde mit Sauerstoff in Gegenwart von Lithium- oder Erdalkalimetallverbindungen, die in Mengen von 0,01 bis 10 Gew.-%, bezogen auf das ganze Reaktionssystem, eingesetzt werden, um aliphatische, α-verzweigte Carbonsäuren herzustellen. Auch nach dem in der französischen Patentanmeldung 2 769 624 beschriebenen Tieftemperaturoxidationsverfahren wird in Gegenwart von Alkali- bzw. Erdalkalimetallverbindungen als Zusatzstoffe gearbeitet. DE-C1-100 10 771 offenbart bei der Oxidation von 2-Methylbutanal sowohl die alleinige Verwendung von Alkalisalzen als auch im Gemisch mit Übergangsmetallen.

Aber auch bei der Oxidation von aliphatischen geradkettigen oder verzweigten Aldehyden, die in α-Position keine Alkylverzweigung tragen, wird die Verwendung eines Gemisches aus Alkali- oder Erdalkalimetallcarboxylaten mit Übergangsmetallen beschrieben. DE 10 2004 055 252 A1 offenbart die Oxidation von n-Pentanal oder von Isononanal auf Basis von 3,5,5-Trimethylhexanal in Gegenwart des entsprechenden Kaliumcarboxylates und Eisen. Die nach Oxidation erhaltene Rohsäure wird destillativ abgetrennt und der anfallende metallhaltige Destillationsrückstand kann erneut in die Aldehydoxidation eingesetzt werden. Nach der Lehre von DE 10 2006 022 168 A1 wird ein Gemisch aus Alkali- oder Erdalkalimetallcarboxylaten und Übergangsmetallen in einer ersten Aldehydoxidationsreaktion als Umsetzungsprodukt hergestellt und dieses Umsetzungsprodukt für die nachfolgende Oxidation von aliphatischen geradkettigen oder β-alkylverzweigten Monocarbonsäuren wieder eingesetzt.

Üblicherweise wird zunächst in einer separaten Reaktion die jeweilige Carbonsäure mit einer wässrigen Lösung einer Alkali- oder Erdalkalimetallverbindung, vorzugsweise mit einer wässrigen Alkali- oder Erdalkalimetallhydroxidlösung in das jeweilige Carboxylat überführt, das dem zu oxidierenden Aldehyd zugemischt wird. Auch kann dem Reaktionsgemisch eine wässrige Alkali- oder Erdalkalimetallhydroxidlösung zugesetzt werden, so dass die Bildung der jeweiligen Carboxylate während der Oxidation erfolgt. Insbesondere hat sich der Einsatz von Kaliumcarboxylaten bewährt. Nach erfolgter Oxidationsreaktion wird die Rohsäure destillativ aufgearbeitet, wobei ein hochviskoser, alkali- oder erdalkalimetallcarboxylathaltiger Destillationsrückstand anfällt. Dieser Destillationsrückstand kann bis zu einem bestimmten Grade wieder in den Oxidationsprozess zurückgeführt werden. Mit steigender Rückführungsrate nimmt jedoch die Selektivität der Aldehydoxidation ab, so dass letzteridlich der Destillationsrückstand aus dem Verfahren ausgeschleust werden muss.

Da der hochviskose Destillationsrückstand neben Hochsiedern jedoch in überwiegendem Maße die gewünschte aliphatische Monocarbonsäure enthält, entweder in Form des Carboxylates oder als physikalisch eingemischte freie aliphatische Monocarbonsäure, ist es wünschenswert, die aliphatische Monocarbonsäure auf einfache Weise aus den Rückständen der Monocarbonsäuredestillation zurückzugewinnen und so die Produktausbringung und damit die Wirtschaftlichkeit des Oxidationsverfahrens zu verbessern. Auch soll der Anfall des hochviskosen Destillationsrückstandes verringert werden, um den Entsorgungsaufwand zu reduzieren.

Die vorliegende Erfindung besteht daher in einem Verfahren zur Gewinnung von aliphatischen Monocarbonsäuren mit 4 bis 11 Kohlenstoffatomen aus dem Destillationsrückstand, der bei der Oxidation der entsprechenden Aldehyde mit Sauerstoff oder sauerstoffenthaltenden Gasgemischen in Gegenwart von Alkali- oder Erdalkalimetallcarboxylaten zur entsprechenden Monocarbonsäure und nachfolgender Destillation anfällt, dadurch gekennzeichnet, dass der Destillationsrückstand mit einer wässrigen Säure in einem Rohrreaktor in Kontakt gebracht wird und das aus dem Rohrreaktor abfließende Zweiphasengemisch in einen Absetzbehälter geleitet wird, in dem die sich scheidende organische Phase einen pH-Wert von 4,5 oder kleiner aufweist.

Überraschenderweise trennt sich das nach dem Verlassen des Rohrreaktors vorliegende Zweiphasengemisch aus dem behandelten organischen Destillationsrückstand und der wässrigen Säure in einem nachgeschalteten Absetzbehälter unproblematisch in die flüssige organische und wässrige Phase. Die Phasentrennung erfolgt spontan und scharf ohne Bildung einer schaumartigen Zwischenschicht. Eine derartige vorteilhafte scharfe Phasentrennung war nicht zu erwarten, da man aufgrund der oberflächenaktiven Eigenschaften der Alkali- oder Erdalkalimetallcarboxylate bei Kontakt mit der wässrigen Phase mit einer Schaumbildung hätte rechnen können. Die rasche und scharfe Phasentrennung ermöglicht auf der einen Seite einen hohen Durchsatz mit dem organischen Destillationsrückstand und der wässrigen Säure. Weiterhin hält sich nach Phasentrennung die Belastung der wässrigen Phase mit organischen Bestandteilen in Grenzen und der Alkali- oder Erdalkalimetallgehalt in der organischen Phase kann auf ein vertretbares Niveau abgereichert werden.

Als Einsatzmaterial verwendet man den alkali- oder erdalkalimetallhaltigen Rückstand aus der Destillation aliphatischer Monocarbonsäuren mit 4 bis 11 Kohlenstoffatomen, die durch Oxidation der entsprechenden Aldehyde mit Sauerstoff oder sauerstoffenthaltenden Gasgemischen in Gegenwart von Alkali- oder Erdalkalimetallcarboxylaten hergestellt werden. Bei den Alkali- oder Erdalkalimetallcarboxylaten handelt es sich um die Carboxylate beispielsweise des Lithiums, Natriums oder Kaliums sowie des Calciums oder Bariums. Vorzugsweise wird in Gegenwart des entsprechenden Kaliumcarboxylats die Aldehydoxidation durchgeführt. Im Allgemeinen stellt man eine Alkali- oder Erdalkalimetallcarboxylat enthaltende Lösung durch Neutralisation einer wässrigen die Alkali- oder Erdalkalimetallverbindung enthaltenden Lösung mit einem Überschuss an der jeweils gewünschten Carbonsäure her und setzt diese Lösung dem zu oxidierenden aliphatischen Aldehyd zu. Als Alkali- oder Erdalkalimetallverbindungen eignen sich hierzu besonders die Hydroxide, Carbonate oder Hydrogencarbonate. Es ist aber auch möglich, die Alkali- oder Erdalkalimetallcarboxylate im Reaktionsgemisch zu erzeugen, indem man dem Reaktionsgemisch Alkali- oder Erdalkalimetallverbindungen zusetzt, die unter den Reaktionsbedingungen in die Carboxylate überführt werden. Beispielsweise lassen sich Alkali- oder Erdalkalimetallhydroxide, -carbonate, -hydrogen-carbonate oder -oxide verwenden. Ihr Zusatz kann entweder in fester Form oder als wässrige Lösung erfolgen.

Der Alkali- oder Erdalkalimetallgehalt im aufzuarbeitenden Destillationsrückstand liegt im Allgemeinen in einem Bereich von 3 bis 15 Gew.-%, vorzugsweise von 5 bis 10 Gew.-%, bezogen auf den gesamten Destillationsrückstand. Neben dem entsprechend gebundenen Carboxylat enthält der Destillationsrückstand auch noch freie aliphatische Monocarbonsäure in Abhängigkeit von den Destillationsbedingungen. Der organische Anteil des Destillationsrückstands besteht, je nach Destillationsbedingungen, bis zu 98 Gew.-% aus der entsprechenden aliphatischen Monocarbonsäure, in Form von freier aliphatischer Monocarbonsäure und des entsprechenden Carboxylates. Der Rest auf 100% im organischen Anteil enthält überwiegend sauerstoffhaltige Hochsieder. Die angegebene Zusammensetzung kann als Richtwert angesehen werden und kann durch die Destillationsbedingungen, beispielsweise durch den Eindickungsgrad, variabel eingestellt werden. Eine zu hohe Aufkonzentration ist jedoch zu vermeiden, da ansonsten der aufzuarbeitende Destillationsrückstand eine zu hohe Viskosität aufweist und nicht mehr zufriedenstellend umgepumpt werden kann. Aufgrund der hochviskosen Konsistenz empfiehlt es sich, den Destillationsrückstand vor Aufgabe in den Rohrreaktor auf eine Temperatur von 30 bis 90°C, vorzugsweise 50 bis 80°C vorzuheizen.

Der aufzuarbeitende Destillationsrückstand wird mit einer wässrigen Säure in dem Rohrreaktor in Kontakt gebracht. Dabei kann der organische und wässrige Stoffstrom getrennt aber gleichzeitig in den Rohrreaktor eingeleitet werden. Vorzugsweise werden beide Flüssigkeiten vorab gemischt und als Zweiphasengemisch aus organischer und wässriger Phase auf den Rohrreaktor gegeben. In einer besonders bevorzugten Ausführungsform wird das Zweiphasengemisch vor Eintritt in den Rohrrektor durch ein vorgeschaltetes statisches Mischelement geführt, um den Kontakt zwischen beiden Phasen zu intensivieren. Solche Mischelemente stehen kommerziell zur Verfügung und werden beispielsweise als Sulzermischer oder Kenicksmischer angeboten mit speziellen Produktlinien für das Vermischen unterschiedlich viskoser Flüssigkeiten.

Der aufzuarbeitende Destillationsrückstand und die wässrige Säure können getrennt oder als Gemisch auf den Rohrreaktor aufgegeben werden. Bei der getrennten Zugabe können organische und wässrige Phase entweder im Gleichstrom oder im Gegenstrom in den Rohrreaktor einfließen. Als Rohrreaktor eignet sich beispielsweise ein beliebig angeordnetes Strömungsrohr, wie ein senkrecht stehendes oder ein waagerecht angeordnetes Strömungsrohr oder ein mehrfach geschlängeltes Strömungsrohr. Der Rohrreaktor kann ebenfalls Füllkörper oder Einbauten enthalten, beispielsweise Raschigringe, Sättel, Pallringe, Wendel, Strombrecher oder statische Mischer oder Mischerpackungen. Vorzugsweise wird der Reaktor kontinuierlich betrieben.

Als wässrige Säure werden wässrige Lösungen anorganischer Säuren eingesetzt, die eine ausreichende Säurestärke besitzen, um die im aufzuarbeitenden Destillationsrückstand enthaltenen Alkali- oder Erdalkalimetallcarboxylate in die entsprechenden aliphatischen Monocarbonsäuren umzusalzen. Als anorganische Säuren eignen sich beispielsweise Salzsäure, Schwefelsäure, Phosphorsäure oder Salpetersäure, die als wässrige Lösung mit einem Säuregehalt von 1 bis 20 Gew.-%, vorzugsweise 5 bis 10 Gew.-% eingesetzt werden. Besonders bewährt sich eine wässrige 5 bis 10 Gew.-%ige Schwefelsäurelösung. Die wässrige Säure wird in einer solchen Menge eingesetzt, dass pro Equivalent Alkali- oder Erdalkalimetallcarboxylat ein bis zu 20%-iger, vorzugsweise 10%-iger equivalenter Überschuss, bezogen auf die zur vollständigen Umsalzung erforderlichen Säuremenge, vorliegt. Die Umsalzung oder Freisetzung der aliphatischen Monocarbonsäure erfolgt aufgrund der hohen Viskosität des Destillationsrückstands vorzugsweise bei einer Temperatur von 30 bis 90°C, insbesondere 50 bis 80°C unter Eigendruck oder leichtem Überdruck, obwohl die Anwendung höherer Drücke, beispielsweise bis 0,8 MPa, nicht ausgeschlossen ist.

Es hat sich eine Belastung des Rohrreaktors V/Vh mit dem aufzuarbeitenden Destillationssumpf von 0,1 bis 10 h⁻¹ und mit der zugeführten wässrigen Säure von 0,5 bis 25 h⁻¹, jeweils bezogen auf das Reaktorvolumen und Zeit, als zweckmäßig erwiesen, wenn beide Stoffströme getrennt aber gleichzeitig in den Rohrreaktor eingespeist werden. Wird zuvor der Destillationssumpf und die wässrige Säure vermischt, vorzugsweise über ein dem Rohrreaktor vorgeschaltetes statisches Mischelement, dann gibt man das heterogene Zweiphasengemisch mit einer Belastung von 0,5 bis 35 h⁻¹ pro Reaktorvolumen und Zeit auf den Rohrreaktor. Die Belastung kann über einen weiten Bereich variiert werden und selbst bei hohen Reaktorbelastungen im Bereich von 20 bis 32 h⁻¹ beobachtet man eine ausreichende Freisetzung der gewünschten aliphatischen Monocarbonsäure und dementsprechend eine Abreicherung des Alkali- oder Erdalkalimetallgehalts in der organischen Phase.

Das aus dem Rohrreaktor abfließende Zweiphasengemisch wird in einen Absetzbehälter geleitet, in dem sich spontan die wässrige von der organischen Phase scheidet. Die Phasentrennung ist scharf und ohne Bildung einer schaumartigen Zwischenschicht. Der in der organischen Phase ermittelte pH-Wert korreliert mit dem Restgehalt an Alkali- oder Erdalkalimetall und weist 4,5 pH-Einheiten oder kleiner auf. Unterhalb dieses pH-Wertes wird ein ausreichend geringer Alkali- oder Erdalkalimetallgehalt von weniger als 1000 ppm in der organischen Phase sichergestellt. Sollte der pH-Wert in der organischen Phase oberhalb von 4,5 liegen, sind die Reaktionsbedingungen, wie die verwendete Säuremenge und -konzentration und die Belastung des Rohrreaktors mit der wässrigen Säure so zu variieren, dass die organische Phase nach Phasentrennung einen pH-Wert von 4,5 oder weniger aufweist. Die gefundene Korrelation zwischen dem pH-Wert in der organischen Phase und dem Alkali- oder Erdalkalimetallgehalt gestattet eine einfache Überwachung des Umsalzungsprozesses, da pH-Werte messtechnisch sehr einfach auch im laufenden Verfahren überwacht werden können. Die obere organische Phase besteht im Wesentlichen aus der durch die Umsalzung freigesetzten aliphatischen Monocarbonsäure, die in nachgeschalteten Destillationseinrichtungen weiter aufgearbeitet wird. Um in der nachgeschalteten Reindestillation Zersetzungsprozesse und die Bildung farbgebender Komponenten zu vermeiden, ist ein Restgehalt an Alkali- oder Erdalkalimetallsalzen von weniger als 1000 ppm empfehlenswert.

Die im Absetzbehälter anfallende wässrige Lösung enthält das Alkali- oder Erdalkalimetallsalz der entsprechenden anorganischen Säure sowie die zur Umsalzung im Überschuss zugegebene anorganische Säure. Die wässrige Phase, die einen pH-Wert im Bereich von 0,2 bis 1,8 aufweist, wird als Abwasser aus dem Prozess entfernt und kann zur Verringerung des Gehalts an organischen Verunreinigungen mit einem polaren organischen Lösungsmittel, beispielsweise mit Alkoholen, Estern oder Ethern extrahiert werden. Es ist aber auch möglich, die abgetrennte wässrige Phase unter Zugabe von Frischsäurelösung wieder in den Rohrreaktor zurückzuführen.
Die nach dem erfindungsgemäßen Verfahren aus Destillationsrückständen zurückgewonnenen aliphatischen Monocarbonsäuren mit 4 bis 11 Kohlenstoffatomen werden durch Oxidation mit Sauerstoff oder sauerstoffenthaltenden Gasen der entsprechenden C₄- bis C₁₁-Aldehyden erhalten. Die Herkunft der Aldehyde ist nicht auf bestimmte Herstellungsverfahren beschränkt. Aufgrund ihrer einfachen Zugänglichkeit werden durch Oxosynthese, d.h. durch Reaktion von C₃- bis C₁₀-Olefinen mit Kohlenmonoxid und Wasserstoff gewonnene Aldehyde bevorzugt. In diesem Zusammenhang ist es nicht entscheidend, welche spezielle Ausführungsform der Oxosynthese zur Gewinnung der Aldehyde diente, d.h. ob die Reaktion z.B. durch Kobalt oder durch Rhodium katalysiert wurde, ob man die Metalle allein oder zusammen mit Komplexbildnern einsetzte und der Katalysator im Reaktionsgemisch homogen gelöst war oder eine eigene, heterogene Phase bildete. Das erfindungsgemäße Verfahren eignet sich für aliphatische geradkettige oder verzweigte Monocarbonsäuren.

Besonders eignet sich das erfindungsgemäße Verfahren zur Rückgewinnung von aliphatischen, α-alkylverzweigten Monocarbonsäuren mit 4 bis 11 Kohlenstoffatomen, da zur Selektivitätsverbesserung üblicherweise die Oxidation in Gegenwart von Alkali- oder Erdalkalimetallcarboxylaten erfolgt. Insbesondere lassen sich iso-Buttersäure, 2-Ethylbuttersäure, 2-Methylbuttersäure, 2-Methylpentansäure, 2-Methylhexansäure, 2-Ethylhexansäure, 2-Methyloctansäure, 2-Methylnonansäure sowie 2-Propylheptansäure aus den Destillationsrückständen zurückgewinnen. Das erfindungsgemäße Verfahren kann aber auch mit Erfolg auf die Rückgewinnung geradkettiger oder beliebig verzweigter aliphatischer Monocarbonsäuren angewandt werden, die keine α-Alkylverzweigung tragen, sofern die Aldehydoxidation in Gegenwart von Alkali- oder Erdalkalimetallcarboxylaten erfolgt. Beispielhaft lassen sich n-Buttersäure, n-Pentansäure, n-Hexansäure, n-Heptansäure, n-Octansäure, n-Nonansäure, n-Decansäure, beliebig verzweigte iso-Pentansäure, beliebig verzweigte iso-Hexansäure, beliebig verzweigte iso-Heptansäure, beliebig verzweigte iso-Octansäure, beliebig verzweigte iso-Nonansäure, beliebig verzweigte iso-Decansäure oder beliebig verzweigte iso-Undecansäure nach dem erfindungsgemäßen Verfahren zurückgewinnen. Als beliebig verzweigte iso-Nonansäure kann besonders vorteilhaft iso-Nonansäure mit dem Hauptbestandteil 3,5,5-Trimethylhexansäure mit der CAS-Nummer 3302-10-1, wobei der entsprechende Aldehyd durch Oxosynthese von Di-isobutylen zugänglich ist, zurückgewonnen werden. Als beliebig verzweigte iso-Pentansäure kann 3-Methylbuttersäure zurückgewonnen werden. Das erfindungsgemäße Verfahren kann ebenfalls auf die Rückgewinnung ungesättigter, aliphatischer Monocarbonsäuren, wie zum Beispiel auf ungesättigte aliphatische, α-alkylverzweigte Monocarbonsäuren wie 2-Ethylbutensäure, 2-Methylpentensäure, 2-Ethylhexensäure und 2-Propylheptensäure ausgedehnt werden, obwohl die Aufarbeitung von Destillationsrückständen aus der Herstellung dieser Typen von Monocarbonsäuren eher auf Sonderfälle beschränkt bleibt.

Durch die Gewinnung der gewünschten aliphatischen Monocarbonsäure aus den alkali- oder erdalkalimetallhaltigen Destillationsrückständen kann die Wirtschaftlichkeit des gesamten Herstellprozesses für aliphatische Monocarbonsäuren und ihre Ausbeuten deutlich verbessert werden.

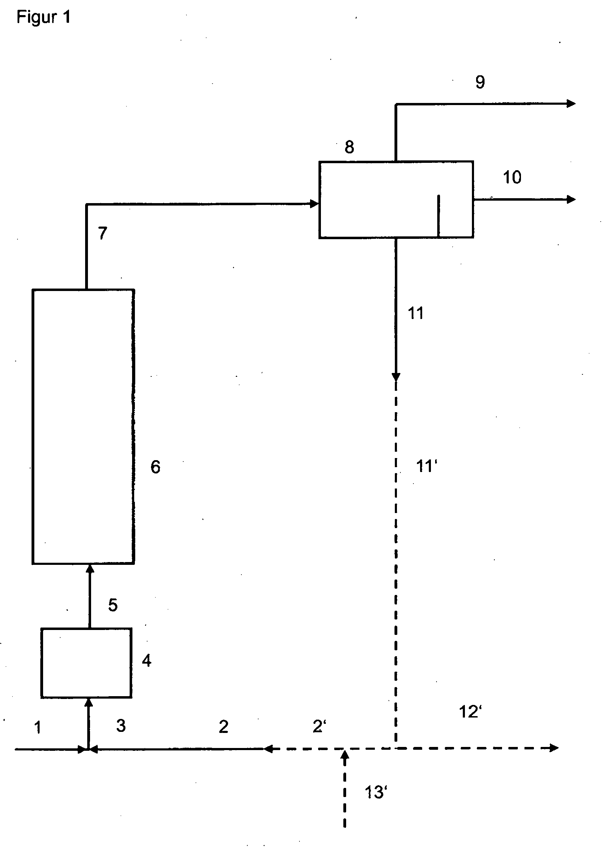

Das erfindungsgemäße Verfahren wird im Folgenden an Hand des Prinzipschemas gemäß Figur 1 näher erläutert. Das erfindungsgemäße Verfahren ist aber nicht auf die in der Zeichnung dargestellte Ausführungsform beschränkt.

Über Leitung (1) werden der auf 50 bis 80°C erwärmte alkali- oder erdalkalimetallhaltige Rückstand aus der Destillation aliphatischer Monocarbonsäuren und über Leitung (2) eine verdünnte wässrige Lösung einer anorganischen Säure herangeführt und nach dem Zusammenführen über Leitung (3) in dem statischen Mischer (4) intensiv vermischt. Das Zweiphasengemisch tritt anschließend über Leitung (5) in den Boden des Rohrreaktors (6) ein. Am Reaktorkopf wird über die Leitung (7) der flüssige Reaktoraustrag abgeführt und in einen Absetzbehälter (8) geleitet, in dem sich die leichtere organische Phase von der schwereren wässrigen Phase trennt. Gasförmige Anteile werden über die Leitung (9) abgeführt. Die abgesetzte organische Phase, die die gewünschte aliphatische Monocarbonsäure enthält, verlässt den Absetzbehälter (8) über die Leitung (10) und wird in nachfolgenden Destillationsschritten zur Reinsäure aufdestilliert (nicht in Figur 1 gezeigt). Die im Absetzbehälter (8) anfallende wässrige Lösung enthält das Alkali- oder Erdalkalisalz der zugesetzten anorganischen Säure und wird über die Leitung (11) abgeführt. Zur Reduzierung des organischen Anteils kann die wässrige Phase noch mit einem polaren organischen Lösungsmittel, beispielsweise mit einem organischen Alkohol wie 2-Ethylhexanol, extrahiert werden.

In einer weiteren Ausgestaltung kann die über Leitung (11) abgeführte wässrige Lösung über die Leitung (11') und über die Leitung (2') wieder in den Prozess zurückgeführt werden, gegebenenfalls nach Entfernung eines Teilstromes über Leitung (12') und Zugabe von Frischsäure über Leitung (13').

Im Folgenden wird das erfindungsgemäße Verfahren anhand einiger Beispiele näher erläutert, es ist jedoch nicht auf die beschriebenen Ausführungsformen beschränkt.

### Beispiele:

### Rückgewinnung von 2-Ethylhexansäure

Zum Einsatz kam ein kaliumhaltiger Destillationsrückstand aus der Oxidation von 2-Ethylhexanal in Gegenwart von Kalium-2-ethylhexanoat zu 2-Ethylhexansäure mit nachfolgender Destillation, der folgende gaschromatographisch ermittelte Zusammensetzung (Gew.-%) aufwies:

| | |
|---|---|
| Vorlauf | 0,5 |
| Zwischenlauf | 0,6 |
| 2-Ethylhexansäure | 96,2 |
| Nachlauf | 2,7 |
| | |
| Kaliumgehalt (Gew.-%) bezogen auf den Destillationsrückstand | 8,7 |

Die Behandlung des kaliumhaltigen Rückstandes aus der Herstellung und Destillation von 2-Ethylhexansäure erfolgte nach dem Versuchsaufbau, der in Figur 1 prinzipiell dargestellt ist. Über Leitung (1) wurde der auf 50°C erwärmte Destillationsrückstand und über Leitung (2) eine ebenfalls auf 50°C erwärmte 5 Gew.-%ige wässrige Schwefelsäure herangeführt. Beide Flüssigkeiten wurden in der Leitung 3 vereinigt und in dem statischen Mischer (4), Sulzermischer Typ SMX DN4 intensiv vermischt. Über die Leitung (5) wurde das Zweiphasengemisch auf den Boden einer senkrecht stehenden Füllkörperkolonne mit 20 cm Länge und einer 250 mL Schüttung aus 2 mm V2A-Wendeln geführt. Das am Reaktorkopf über die Leitung (7) entnommene Zweiphasengemisch gelangte in den Phasentrenner (8), in dem eine spontane Phasentrennung mit scharfen Phasengrenzen innerhalb weniger Sekunden einsetzte. Die freigesetzte rohe 2-Ethylhexansäure wurde über Leitung (10) abgeführt, die untere, wässrige Phase mit überschüssiger Schwefelsäure und Kaliumhydrogensulfat/Kaliumsulfat wurde über Leitung (11) ausgeschleust und gasförmige Anteile über Leitung (9) abgeführt.

In der nachfolgenden Tabelle 1 sind die Ergebnisse aus unterschiedlichen pH-Werteinstellungen zusammengestellt. Dabei zeigt sich, dass nach Phasentrennung in der organischen Phase zwischen dem pH-Wert und dem Kaliumgehalt eine Korrelation besteht, so dass über die einfach durchzuführende pH-Wert-Messung auf den Kaliumgehalt in der organischen Phase geschlossen werden kann. Die pH-Wert-Messung erfolgte mit einem pH-Meter des Typs CG836 der Firma Schott.

**Tabelle 1: Umsetzung der Destillationsrückstände aus der 2-Ethylhexansäureherstellung mit 5%-iger, wässriger Schwefelsäure im Rohrreaktor**

| **Versuch** | **pH-Wert org. Phase** | **pH-Wert wäss. Phase** | **Kaliumgehalt in org. Phase (%)^{(*)}** | **Verhältnis org./ wäss. Phase** | **V/Vh (1/h)^{(***)}** |
|---|---|---|---|---|---|
| 1 | 6,5 | 6,3 | 1,8 | 1,58 | n.b. |
| 2 | 5,9 | 4,8 | 1,4 | 0,52 | 1,7 |
| 3 | 5,7 | 2,9 | 0,67 | 0,74 | n.b. |
| 4 | 5,1 | 0,8 | 0,08 | 0,39 | n.b. |
| 5 | 4,7 | 1,2 | 0,04 | 0,32 | n.b. |
| 6 | 4,3 | 1,3 | u.N. | 0,28 | 1,4 |
| 7 | 4,1 | 1,0 | u.N. | 0,17 | 1,6 |
| 8 | 2,6 | 1,3 | u.N. | 0,23 | 1,5 |

| | | | | | |
|---|---|---|---|---|---|
| ^{(*)}u.N. unterhalb der Nachweisgrenze; ^{(**)} n.b. nicht bestimmt | | | | | |

Wie die Ergebnisse aus Versuch 5 und 6 zeigen, sollte der pH-Wert in der organischen Phase weniger als 4,5 betragen, um eine ausreichend große Kaliumabreicherung sicherzustellen. Die Bestimmung des Kaliumgehalts erfolgte durch Titration des Kalium-2-ethylhexanoates mit Salzsäure und Umrechnung auf Kalium mit einer Nachweisgrenze von 100 ppm. Wenn man den pH-Wert der wässrigen Phase zur Orientierung für den Kaliumgehalt in der organischen Phase heranzieht, sollte ein pH-Wert von kleiner 1,3 eingestellt werden.

### Wiedereinsatz der wässrigen Phase:

Die Versuchsbedingung des Versuches 7 wurde dahingehend modifiziert, dass die im Phasentrenner (8) abgeschiedene wässrige, saure Lösung über die Leitungen (11') und (2') ohne Frischsäurezugabe wieder in den Neutralisationsprozess zurückgeführt wurde. Die Ergebnisse sind in der nachfolgenden Tabelle 2 aufgeführt.

**Tabelle 2: Wiedereinsatz der wässrigen Phase für die Umsetzung der Destillationsrückstände aus der 2-Ethylhexansäureherstellung**

| Versuch | pH-Wert org. Phase | pH-Wert wäss. Phase | Kaliumgehalt in org. Phase (%) | Verhältnis org./wäss. Phase | V/Vh (1/h) |
|---|---|---|---|---|---|
| 7 | 4,1 | 1,0 | u.N. | 0,17 | 1,6 |
| 7(a) | 4,3 | 1,1 | u.N. | 0,22 | 1,6 |

Auch der Wiedereinsatz in Versuch 7(a) belegt, dass man sich an den pH-Wertvorgaben für die organische Phase richten kann, um auf den Kaliumgehalt in der organischen Phase zu schließen.

### Variation der Reaktorbelastung:

In den folgenden Versuchen wurde das Volumen der Füllkörperkolonne verringert und auf diese Weise ihre Belastung erhöht. Zum Einsatz kam eine Füllkörperkolonne mit einer Länge von 40 cm und einem Durchmesser von 10 mm, die 2 mm V2A-Wendel mit einer Füllhöhe von 30 cm als Schüttung enthielt.

**Tabelle 3: Umsetzung der Destillationsrückstände aus der 2-Ethylhexansäureherstellung mit 5%-iger, wässriger Schwefelsäure im Rohrreaktor unter Variation der Belastung**

| Versuch | pH-Wert org. Phase | pH-Wert wäss. Phase | Kaliumgehalt in org. Phase (%) | Verhältnis org./wäss. Phase | V/Vh (1/h) |
|---|---|---|---|---|---|
| 9 | 5,0 | 0,9 | 0,22 | 0,36 | 27 |
| 10 | 4,9 | 1,6 | 0,22 | 0,41 | 21 |
| 11 | 3,2 | 0,2 | 0,06 | 0,26 | 29 |

Auch die Versuche 9 bis 11 belegen, dass der pH-Wert in der organischen Phase einen direkten Schluss auf den Kaliumgehalt zulässt. Wird der pH-Wert in der organischen Phase ausreichend erniedrigt, wird selbst bei einer hohen Reaktorbelastung eine ausreichende Kaliumabtrennung in der organischen Phase erreicht.

### Rückgewinnung von 2-Methylbuttersäure

Zum Einsatz kam ein kaliumhaltiger Destillationsrückstand aus der Oxidation von 2-Methylbutanal in Gegenwart von Kalium-2-methylbutanoat zu 2-Methylbuttersäure mit nachfolgender Destillation, der folgende gaschromatographisch ermittelte Zusammensetzung (Gew.-%) aufwies:

| | |
|---|---|
| Vorlauf | 0,7 |
| 2-Methylbuttersäure | 62,6 |
| Nachlauf | 36,7 |
| | |
| Kaliumgehalt (Gew.-%) bezogen auf den Destillationsrückstand | 4,1 |

Die Behandlung des kaliumhaltigen Rückstandes aus der Herstellung und Destillation von 2-Methylbuttersäure erfolgte nach dem Versuchsaufbau, der in Figur 1 prinzipiell dargestellt ist. Über Leitung (1) wurde der Destillationsrückstand und über Leitung (2) eine 5 Gew.-%ige wässrige Schwefelsäure herangeführt. Beide Flüssigkeiten wurden in der Leitung 3 vereinigt und in dem statischen Mischer (4), Sulzermischer Typ SMX DN4 intensiv vermischt. Über die Leitung (5) wurde das Zweiphasengemisch auf den Boden einer senkrecht stehenden Füllkörperkolonne mit einer Länge von 40 cm und einem Durchmesser von 10 mm, die 2 mm V2A-Wendel mit einer Füllhöhe von 30 cm als Schüttung enthielt, geführt. Das am Reaktorkopf über die Leitung (7) entnommene Zweiphasengemisch gelangte in den Phasentrenner (8), in dem eine spontane Phasentrennung mit scharfen Phasengrenzen innerhalb weniger Sekunden einsetzte. Die freigesetzte rohe 2-Methylbuttersäure wurde über Leitung (10) abgeführt, die untere, wässrige Phase mit überschüssiger Schwefelsäure und Kaliumhydrogensulfat/Kaliumsulfat wurde über Leitung (11) ausgeschleust und gasförmige Anteile über Leitung (9) abgeführt.

In der nachfolgenden Tabelle 4 sind die Ergebnisse aus unterschiedlichen pH-Werteinstellungen zusammengestellt. Dabei zeigt sich, dass nach Phasentrennung in der organischen Phase zwischen dem pH-Wert und dem Kaliumgehalt eine Korrelation besteht, so dass über die einfach durchzuführende pH-Wert-Messung auf den Kaliumgehalt in der organischen Phase geschlossen werden kann. Die pH-Wert-Messung erfolgte mit einem pH-Meter des Typs CG836 der Firma Schott.

**Tabelle 4: Umsetzung der Destillationsrückstände aus der 2-Methylbuttersäureherstellung mit 5%-iger, wässriger Schwefelsäure im Rohrreaktor**

| **Versuch** | **pH-Wert org. Phase** | **pH-Wert wäss. Phase** | **Kaliumgehalt in org. Phase (%)** | **Verhältnis org./ wäss. Phase** | **V/Vh (1/h)** |
|---|---|---|---|---|---|
| 12 | 4,2 | 2,9 | 0,44 | 0,96 | 16 |
| 13 | 2,6 | 1,6 | 0,05 | 1,12 | 23 |
| 14 | 2,5 | 1,3 | 0,03 | 0,66 | 18 |
| 15 | 2,6 | 1,0 | 0,01 | 0,50 | 18 |

Wie die Ergebnisse aus Versuch 12 und 13 zeigen, sollte der pH-Wert in der organischen Phase weniger als 4,0 betragen, um eine ausreichend große Kaliumabreicherung sicherzustellen. Die Bestimmung des Kaliumgehalts erfolgte durch Titration des Kalium-2-methylbutanoates mit Salzsäure und Umrechnung auf Kalium mit einer Nachweisgrenze von 100 ppm. Wenn man den pH-Wert der wässrigen Phase zur Orientierung für den Kaliumgehalt in der organischen Phase heranzieht, sollte ein pH-Wert von kleiner 1,6 eingestellt werden.

### Wiedereinsatz der wässrigen Phase:

Die Versuchsbedingung des Versuches 14 wurde dahingehend modifiziert, dass die im Phasentrenner (8) abgeschiedene wässrige, saure Lösung über die Leitungen (11') und (2') ohne Frischsäurezugabe wieder in den Neutralisationsprozess zurückgeführt wurde. Die Ergebnisse sind in der nachfolgenden Tabelle 5 aufgeführt.

**Tabelle 5: Wiedereinsatz der wässrigen Phase für die Umsetzung der Destillationsrückstände aus der 2-Methylbuttersäureherstellung**

| Versuch | pH-Wert org. Phase | pH-Wert wäss. Phase | Kaliumgehalt in org. Phase (%) | Verhältnis org./wäss. Phase | V/Vh (1/h) |
|---|---|---|---|---|---|
| 14 | 2,5 | 1,3 | 0,03 | 0,66 | 18 |
| 14 (a) | 3,6 | 1,9 | 0,25 | 0,43 | 17 |
| 14 (b) | 4,6 | 4,8 | 1,4 | 0,84 | 20 |

Auch die Wiedereinsätze in den Versuchen 14(a) und 14(b) belegen, dass man sich an den pH-Wertvorgaben für die organische Phase richten kann, um auf den Kaliumgehalt in der organischen Phase zu schließen.

## Patentansprüche

1. Verfahren zur Gewinnung von aliphatischen Monocarbonsäuren mit 4 bis 11 Kohlenstoffatomen aus dem Destillationsrückstand, der bei der Oxidation der entsprechenden Aldehyde mit Sauerstoff oder Sauerstoff enthaltenden Gasgemischen in Gegenwart von Alkali- oder Erdalkalimetallcarboxylaten zur entsprechenden Monocarbonsäure und nachfolgender Destillation anfällt, **dadurch gekennzeichnet, dass** der Destillationsrückstand mit einer wässrigen Säure in einem Rohrreaktor zur Reaktion gebracht wird und das aus dem Rohrreaktor abfließende Zweiphasengemisch in einen Absetzbehälter geleitet wird, in dem die sich scheidende organische Phase einen pH-Wert von 4,5 oder kleiner aufweist.

2. Verfahren gemäß Anspruch 1, **dadurch gekennzeichnet, dass** dem Rohrreaktor ein statisches Mischelement vorgeschaltet ist.

3. Verfahren gemäß Anspruch 1 oder 2, **dadurch gekennzeichnet, dass** der Rohrreaktor Füllkörper oder Einbauten enthält.

4. Verfahren nach einem oder mehreren der Ansprüche 1 bis 3, **dadurch gekennzeichnet, dass** der Destillationsrückstand auf eine Temperatur von 30 bis 90°C, vorzugsweise 50 bis 80°C, vorgeheizt wird.

5. Verfahren nach einem oder mehreren der Ansprüche 1 bis 4, **dadurch gekennzeichnet, dass** die Reaktion bei einer Temperatur von 30 bis 90°C, vorzugsweise von 50 bis 80°C erfolgt.

6. Verfahren nach einem oder mehreren der Ansprüche 1 bis 5, **dadurch gekennzeichnet, dass** man als wässrige Säure eine wässrige Lösung einer anorganischen Säure verwendet.

7. Verfahren nach Anspruch 6, **dadurch gekennzeichnet, dass** man als anorganische Säure Salzsäure, Schwefelsäure, Phosphorsäure oder Salpetersäure verwendet.

8. Verfahren nach einem oder mehreren der Ansprüche 1 bis 7, **dadurch gekennzeichnet, dass** man die Destillationsrückstände aus der Herstellung von aliphatischen geradkettigen oder verzweigten Monocarbonsäuren verwendet.

9. Verfahren gemäß Anspruch 8, **dadurch gekennzeichnet, dass** man die Destillationsrückstände aus der Herstellung von aliphatischen geradkettigen Monocarbonsäuren ausgewählt aus der Gruppe von n-Buttersäure, n-Pentansäure, n-Hexansäure, n-Heptansäure, n-Octansäure, n-Nonansäure und n-Decansäure verwendet.

10. Verfahren gemäß Anspruch 8, **dadurch gekennzeichnet, dass** man die Destillationsrückstände aus der Herstellung von aliphatischen, α-alkylverzweigten Monocarbonsäuren verwendet.

11. Verfahren gemäß Anspruch 10, **dadurch gekennzeichnet, dass** man als aliphatische, α-alkylverzweigte Monocarbonsäure iso-Buttersäure, 2-Ethylbuttersäure, 2-Methylbuttersäure, 2-Methylpentansäure, 2-Methylhexansäure, 2-Ethylhexansäure, 2-Methyloctansäure, 2-Methylnonansäure, 2-Propylheptansäure, 2-Ethylbutensäure, 2-Methylpentensäure, 2-Ethylhexensäure oder 2-Propylheptensäure verwendet.

12. Verfahren gemäß Anspruch 8, **dadurch gekennzeichnet, dass** man die Destillationsrückstände aus der Herstellung von aliphatischen verzweigten Monocarbonsäuren verwendet, die nicht α-alkylverzweigt sind.

13. Verfahren gemäß Anspruch 12, **dadurch gekennzeichnet, dass** man als aliphatische verzweigte Monocarbonsäure eine beliebig verzweigte iso-Pentansäure, beliebig verzweigte iso-Hexansäure, beliebig verzweigte iso-Heptansäure, beliebig verzweigte iso-Octansäure, beliebig verzweigte iso-Nonansäure, beliebig verzweigte iso-Decansäure oder beliebig verzweigte iso-Undecansäure verwendet, mit der Maßgabe, dass keine α-Alkylverzweigung vorliegt.

14. Verfahren gemäß Anspruch 13, **dadurch gekennzeichnet, dass** man als beliebig verzweigte iso-Pentansäure 3-Methylbuttersäure verwendet.

15. Verfahren gemäß Anspruch 13, **dadurch gekennzeichnet, dass** man als beliebig verzweigte iso-Nonansäure eine iso-Nonansäure mit dem Hauptbestandteil 3,5,5-Trimethylhexansäure verwendet.

16. Verfahren nach einem oder mehreren der Ansprüche 1 bis 15, **dadurch gekennzeichnet, dass** man die Kalium- oder Natriumcarboxylate enthaltenden Destillationsrückstände der entsprechenden Monocarbonsäuren mit der wässrigen Säure zur Reaktion bringt.

## Claims

1. Process for recovering aliphatic monocarboxylic acids having from 4 to 11 carbon atoms from the distillation residue obtained in the oxidation of the corresponding aldehyde by means of oxygen or oxygen-containing gas mixtures in the presence of alkali metal carboxylates and alkaline earth metal carboxylates to form the corresponding monocarboxylic acid and subsequent distillation, **characterized in that** the distillation residue is reacted with an aqueous acid in a tube reactor and the two-phase mixture flowing out from the tube reactor is introduced into a settling vessel in which the organic phase which separates out has a pH of 4.5 or less.

2. Process according to Claim 1, **characterized in that** a static mixing element is installed upstream of the tube reactor.

3. Process according to Claim 1 or 2, **characterized in that** the tube reactor contains packing elements or internals.

4. Process according to one or more of Claims 1 to 3, **characterized in that** the distillation residue is preheated to a temperature of from 30 to 90°C, preferably from 50 to 80°C.

5. Process according to one or more of Claims 1 to 4, **characterized in that** the reaction is carried out at a temperature of from 30 to 90°C, preferably from 50 to 80°C.

6. Process according to one or more of Claims 1 to 5, **characterized in that** an aqueous solution of an inorganic acid is used as aqueous acid.

7. Process according to Claim 6, **characterized in that** hydrochloric acid, sulphuric acid, phosphoric acid or nitric acid is used as inorganic acid.

8. Process according to one or more of Claims 1 to 7, **characterized in that** the distillation residues from the preparation of aliphatic straight-chain or branched monocarboxylic acids are used.

9. Process according to Claim 8, **characterized in that** the distillation residues from the preparation of aliphatic straight-chain monocarboxylic acids selected from the group consisting of n-butyric acid, n-pentanoic acid, n-hexanoic acid, n-heptanoic acid, n-octanoic acid, n-nonanoic acid and n-decanoic acid are used.

10. Process according to Claim 8, **characterized in that** the distillation residues from the preparation of aliphatic, α-alkyl-branched monocarboxylic acids are used.

11. Process according to Claim 10, **characterized in that** isobutyric acid, 2-ethylbutyric acid, 2-methylbutyric acid, 2-methylpentanoic acid, 2-methylhexanoic acid, 2-ethylhexanoic acid, 2-methyloctanoic acid, 2-methylnonanoic acid, 2-propylheptanoic acid, 2-ethylbutenoic acid, 2-methylpentenoic acid, 2-ethylhexenoic acid or 2-propylheptenoic acid is used as aliphatic, α-alkyl-branched monocarboxylic acid.

12. Process according to Claim 8, **characterized in that** the distillation residues from the preparation of aliphatic branched monocarboxylic acids which are not α-alkyl-branched are used.

13. Process according to Claim 12, **characterized in that** an isopentanoic acid having any branching, isohexanoic acid having any branching, isoheptanoic acid having any branching, isooctanoic acid having any branching, isononanoic acid having any branching, isodecanoic acid having any branching or isoundecanoic acid having any branching is used as aliphatic branched monocarboxylic acid, with the proviso that there is no α-alkyl branching.

14. Process according to Claim 13, **characterized in that** 3-methylbutyric acid is used as isopentanoic acid having any branching.

15. Process according to Claim 13, **characterized in that** an isononanoic acid having the main constituent 3,5,5-trimethylhexanoic acid is used as isononanoic acid having any branching.

16. Process according to one or more of Claims 1 to 15, **characterized in that** the potassium carboxylate- or sodium carboxylate-containing distillation residues of the corresponding monocarboxylic acids are reacted with the aqueous acid.

## Revendications

1. Procédé d'obtention d'acides monocarboxyliques aliphatiques de 4 à 11 atomes de carbone à partir du résidu de distillation formé lors de l'oxydation des aldéhydes correspondants avec de l'oxygène ou des mélanges gazeux contenant de l'oxygène en présence de carboxylates de métaux alcalins ou alcalino-terreux pour former l'acide monocarboxylique correspondant et la distillation ultérieure, **caractérisé en ce que** le résidu de distillation est mis en réaction avec un acide aqueux dans un réacteur tubulaire, et le mélange biphasé déchargé du réacteur tubulaire est introduit dans un contenant de sédimentation dans lequel la phase organique qui se dépose présente un pH de 4,5 ou moins.

2. Procédé selon la revendication 1, **caractérisé en ce que** le réacteur tubulaire est précédé par un élément de mélange statique.

3. Procédé selon la revendication 1 ou 2, **caractérisé en ce que** le réacteur tubulaire contient des corps de remplissage ou des composants intérieurs.

4. Procédé selon une ou plusieurs des revendications 1 à 3, **caractérisé en ce que** le résidu de distillation est préchauffé à une température de 30 à 90 °C, de préférence de 50 à 80 °C.

5. Procédé selon une ou plusieurs des revendications 1 à 4, **caractérisé en ce que** la réaction a lieu à une température de 30 à 90 °C, de préférence de 50 à 80 °C.

6. Procédé selon une ou plusieurs des revendications 1 à 5, **caractérisé en ce qu'**une solution aqueuse d'un acide inorganique est utilisée en tant qu'acide aqueux.

7. Procédé selon la revendication 6, **caractérisé en ce que** l'acide chlorhydrique, l'acide sulfurique, l'acide phosphorique ou l'acide nitrique est utilisé en tant qu'acide inorganique.

8. Procédé selon une ou plusieurs des revendications 1 à 7, **caractérisé en ce que** les résidus de distillation issus de la fabrication d'acides monocarboxyliques aliphatiques linéaires ou ramifiés sont utilisés.

9. Procédé selon la revendication 8, **caractérisé en ce que** les résidus de distillation issus de la fabrication d'acides monocarboxyliques aliphatiques linéaires choisis dans le groupe constitué par l'acide n-butyrique, l'acide n-pentanoïque, l'acide n-hexanoïque, l'acide n-heptanoïque, l'acide n-octanoïque, l'acide n-nonanoïque et l'acide n-décanoïque sont utilisés.

10. Procédé selon la revendication 8, **caractérisé en ce que** les résidus de distillation issus de la fabrication d'acides monocarboxyliques aliphatiques α-alkyl-ramifiés sont utilisés.

11. Procédé selon la revendication 10, **caractérisé en ce que** l'acide iso-butyrique, l'acide 2-éthylbutyrique, l'acide 2-méthylbutyrique, l'acide 2-méthylpentanoïque, l'acide 2-méthylhexanoïque, l'acide 2-éthylhexanoïque, l'acide 2-méthyloctanoïque, l'acide 2-méthylnonanoïque,
l'acide 2-propylheptanoïque, l'acide 2-éthylbuténoïque, l'acide 2-méthylpenténoïque, l'acide 2-éthylhexénoïque ou l'acide 2-propylhepténoïque est utilisé en tant qu'acide monocarboxylique aliphatique α-alkyl-ramifié.

12. Procédé selon la revendication 8, **caractérisé en ce que** les résidus de distillation issus de la fabrication d'acides monocarboxyliques aliphatiques ramifiés qui ne sont pas α-alkyl-ramifiés sont utilisés.

13. Procédé selon la revendication 12, **caractérisé en ce qu'**un acide iso-pentanoïque à ramification quelconque, un acide iso-hexanoïque à ramification quelconque, un acide iso-heptanoïque à ramification quelconque, un acide iso-octanoïque à ramification quelconque, un acide iso-nonanoïque à ramification quelconque, un acide iso-décanoïque à ramification quelconque ou un acide iso-undécanoïque à ramification quelconque est utilisé en tant qu'acide monocarboxylique aliphatique ramifié, à condition qu'aucune ramification α-alkyle ne soit présente.

14. Procédé selon la revendication 13, **caractérisé en ce que** l'acide 3-méthylbutyrique est utilisé en tant qu'acide iso-pentanoïque à ramification quelconque.

15. Procédé selon la revendication 13, **caractérisé en ce qu'**un acide iso-nonanoïque contenant en tant que constituant principal l'acide 3,5,5-triméthylhexanoïque est utilisé en tant qu'acide iso-nonanoïque à ramification quelconque.

16. Procédé selon une ou plusieurs des revendications 1 à 15, **caractérisé en ce que** les résidus de distillation contenant des carboxylates de potassium ou de sodium des acides monocarboxyliques correspondants sont mis en réaction avec l'acide aqueux.
